# EUROPEAN PATENT APPLICATION

(11) **EP 4 497 831 A1**
(43) Date of publication of application: **29.01.2025**
(21) Application number: 23188088.1
(22) Date of filing: 27.07.2023
(51) Int. Cl.: C12Q 1/6804, G01N 33/543

(54) **ANALYSIS ARRAY AND METHOD FOR ANALYSING A BIOLOGICAL SAMPLE**

(71) Applicant: Leica Microsystems CMS GmbH, 35578 Wetzlar (DE)
(72) Inventor: Schlaudraff, Falk, 35578 Wetzlar (DE)
(74) Representative: Schaumburg und Partner Patentanwälte mbB

(57) **Abstract**

An analysis array (100, 100a, 100b) for analysing a biological sample (600) with a target analyte (602, 604, 606) is provided, comprising: a sample area (101) configured to receive the biological sample (600), and a plurality of affinity reagents (200a, 200b, 200c, 300a, 300b, 300c) configured to directly or indirectly bind the target analyte (602, 604, 606). The sample area (101) comprises a plurality of attachment zones (102 ,102a, 102b) for attachment of the affinity reagents (200a, 200b, 200c, 300a, 300b, 300c) to the sample area (101), and each affinity reagent (200a, 200b, 200c, 300a, 300b, 300c) comprises a location oligonucleotide (204, 304a, 304b, 304c, 610). The location oligonucleotide (204, 304a, 304b, 304c, 610) is unique to the attachment zone (102, 102a, 102b) the affinity reagent (200a, 200b, 200c, 300a, 300b, 300c) is attached to. In a further aspect a method for analysing a biological sample (600) with the analysis array (100, 100a, 100b) is provided.

## Description

### Technical field

The invention relates to an analysis array for analysing a biological sample with a target analyte. In a further aspect, a method for analysing the biological sample in a sample area on the analysis array is provided.

### Background

The ability to analyse biological samples, such as tissue sections, by proteomic, genomic and transcriptomic techniques has a rapidly progressed in recent years. The ability to provide super-resolution imaging of these samples has equally seen rapid progress. Yet, the ability to integrate the different types of data and data sources remains a challenge, especially for detecting large numbers of analytes with high reproducibility and high throughput.

For example, providing spatially resolved protein expression profiles in biological samples generally relies on marking the target proteins with optically detectable moieties. In order to detect a large number of target proteins simultaneously in a given sample, the target proteins are marked with distinguishable fluorescent markers. Further, multiplexing approaches are known in the art, which increase the number of proteins that may be distinguished by cyclically staining different sets of target proteins with the fluorescent markers.

However, the use of fluorescent markers inherently limits the number of target analytes that may be detected at any one time. Further, the repeated staining may degrade the sample and consequently be limiting as well. Thus, it is desirable to be able to efficiently detect the presence as well as the location of a large number of target analytes at any one time in a biological sample.

### Summary

It is an object to provide means and a method for efficiently capturing and spatially detecting target analytes in biological samples.

The aforementioned object is achieved by the subject-matter of the independent claims. Advantageous embodiments are defined in the dependent claims and the following description.

An analysis array for analysing a biological sample with a target analyte is provided. The target analyte preferably is an amino acid structure (e.g. a protein) or a lipid. The analysis array comprises: a sample area configured to receive the biological sample, and a plurality of affinity reagents configured to directly or indirectly bind the target analyte. The sample area comprises a plurality of attachment zones for attachment of the affinity reagents to the sample area, and each affinity reagent comprises a location oligonucleotide. The location oligonucleotide is unique to the attachment zone the affinity reagent is attached to. This enables capturing the target analyte on the analysis array and determining the location of target analyte on the analysis array and the biological sample based on a sequencing readout of the location oligonucleotide. Generating an optical readout of markers bound to the target analytes is not necessary and instead, by using a sequencing readout, a larger number of target analytes may be detected at any one time in the biological sample.

In particular, the affinity reagent directly or indirectly binds the target analyte specifically. Thus, the affinity reagent may directly or indirectly only bind the target analyte, for example. As a specific example, the affinity reagent may comprise an antibody that specifically binds to the target analyte.

The target analyte may in particular consist essentially of at least one of a lipid structure, or an amino acid structure. Thus, the target analyte may be a protein of the biological sample, for example. The biological sample may be a tissue section, for example. In particular, a tissue section often comprises a plurality of cells of a certain tissue type with a specific lipid and/or protein expression pattern.

The location oligonucleotide may be a single stranded DNA molecule, for example. In particular, the location oligonucleotide is a unique sequence of nucleotides. The location oligonucleotide may be identified by the unique sequence. Thus, the attachment zone a particular one of the affinity reagents is attached to may be identified by the unique sequence of the location oligonucleotide of that particular affinity reagent. Preferably, the affinity reagents are evenly distributed across the attachment zones.

Preferably, the attachment zones are rectangularly or hexagonally shaped on the sample area with a side length in a range from 5 µm to 100 µm, more preferably in a range from 10 µm to 50 µm, even more preferably in a range from 15 µm to 40 µm. This enables accurately determining the location of target analyte on the analysis array.

The attachment zones may preferably be marked by optically detectable moieties, such asfluorophores, in order to visualise and identify the individual attachment zones in an optical readout, such as an image, of the analysis array.

Preferably, the affinity reagents are attached to the attachment zones via a first linker. The first linker is configured to be cleaved by a first cleavage method, in particular irreversibly cleaved. Thus, cleaving the first linker irreversibly detaches the affinity reagent from the sample area or the attachment zone. This enables efficiently removing the affinity reagents including the location oligonucleotide, the target analyte bound to the affinity reagent, and, in particular, an identity oligonucleotide, from the analysis array.

Preferably, the affinity reagents comprise an amino acid structure or a nucleic acid structure, which is configured to bind the target analyte. This enables specifically binding to a wide range of different target analytes. Preferably, the affinity reagents comprise an amino acid structure in case the affinity reagents are configured to directly bind the target analyte. In case the affinity reagents are configured to indirectly bind the target analyte, preferably the affinity reagents comprise a nucleic acid structure, in particular a capture oligonucleotide. The amino acid structure may be an antibody or an antibody fragment, for example. Further, the amino acid structure may be a peptide-based aptamer. The nucleic acid structure may be a nucleic acid aptamer or an oligonucleotide, for example. Generally, a nucleic acid aptamer has a complex secondary and/or tertiary structure. Thus, the nucleic acid aptamer may bind to the target analyte due to its complex secondary and/or tertiary structure rather than complementary base pairing. This enables specifically binding to a large number of different target analytes.

Preferably, each affinity reagent comprises an identity oligonucleotide unique to the affinity reagent. In particular, this is the case when the affinity reagent is configured to directly bind the target analyte. The identity oligonucleotide is a unique sequence of nucleotides. This enables identifying the affinity reagent and therefore the target analyte that the affinity reagent binds, by the unique sequence of the identity oligonucleotide. In particular, the identity oligonucleotide is unique to the part of the affinity reagent configured to bind the target analyte. Thus, the identity oligonucleotide may be unique to the antibody or aptamer that the affinity reagent comprises. The identity oligonucleotide and the location oligonucleotide of any one affinity reagent are preferably a single nucleotide strand. That single nucleotide strand may be directly attached to the antibody or aptamer, for example.

Preferably, the analysis array comprises a plurality of second affinity reagents configured to bind a second target analyte, wherein each second affinity reagent comprises a second location oligonucleotide unique to the attachment zone the second affinity reagent is attached to, and wherein each second affinity reagent comprises a second identity oligonucleotide unique to the second affinity reagent. This enables capturing several distinct or different target analytes on the analysis array and determining their respective location on the analysis array and the biological sample. Thus, the plurality of second affinity reagents specifically bind the second target analyte. Preferably, the second affinity reagents bind the second target analyte directly and the analysis array further comprises the affinity reagents configured to bind the target analyte directly. In particular, each attachment zone comprises the affinity reagents and the second affinity reagents. Further pluralities of further affinity reagents may be provided, that bind respective further target analytes.

Preferably, the location oligonucleotides are attached to the affinity reagents via a second linker. This enables efficiently removing the location oligonucleotide from the remaining affinity reagent. The second linker is configured to be cleaved by a second cleavage method, in particular irreversibly cleaved. Specifically, the first and second linker may be only cleavable by their respective cleavage method. Thus, the second cleavage method does not cleave the first linker and the first cleavage method does not cleave the second linker. Preferably, the identity oligonucleotide is removed together with the location oligonucleotide upon cleavage of the second linker.

Preferably, the affinity reagents comprise a capture oligonucleotide and the capture oligonucleotide is configured to bind the target analyte via a third affinity reagent configured to directly bind the target analyte of the biological sample, wherein the third affinity reagent comprises an identity oligonucleotide unique to the third affinity reagent, and an oligonucleotide complementary to the capture oligonucleotide of the affinity reagent. This enables flexibly capturing the target analyte on the analysis array and determining its respective location on the analysis array and the biological sample.

In particular, the affinity reagents are configured to indirectly bind the target analyte via the third affinity reagent, which is configured to directly bind the target analyte. Thus, the affinity reagent is configured to bind the third affinity reagent and the third affinity reagent is configured to specifically bind the target analyte. In particular, the sequence of the capture oligonucleotide is unique to provide specific binding of the complementary oligonucleotide of the third affinity reagent and to avoid binding of random oligonucleotides from the biological sample.

Preferably, the third affinity reagents comprise an amino acid structure or a nucleic acid structure, which is configured to directly bind the target analyte. The amino acid structure may be an antibody or an antibody fragment, for example. Further, the amino acid structure may be a peptide-based aptamer. The nucleic acid structure may be a nucleic acid aptamer, for example. Generally, a nucleic acid aptamer has a complex secondary and/or tertiary structure. Thus, the nucleic acid aptamer may bind to the target analyte due to its complex secondary and/or tertiary structure rather than complementary base pairing. Preferably the analysis array comprises at least the third affinity reagent.

The identity oligonucleotide of the third affinity reagent is a unique sequence of nucleotides. This enables identifying the third affinity reagent and therefore the target analyte that the third affinity reagent binds, by the unique sequence of the identity oligonucleotide. In particular, the identity oligonucleotide of the third affinity reagent is unique to the part of the third affinity reagent configured to bind the target analyte. Thus, the identity oligonucleotide may be unique to the antibody or aptamer that the third affinity reagent comprises. The identity oligonucleotide and the capture oligonucleotide of any one third affinity reagent are preferably a single nucleotide strand. That single nucleotide strand may be directly attached to the antibody or aptamer, for example.

In addition, at least a plurality of fourth affinity reagents may be provided, each fourth affinity reagent comprising the complementary oligonucleotide and an identity oligonucleotide unique to the fourth affinity reagent; the fourth affinity reagent being configured to specifically bind a further target analyte.

Preferably, the third affinity reagent comprises the second linker configured to remove the capture oligonucleotide and the identity oligonucleotide from the third affinity reagent.

In a further aspect a method is provided for analysing the biological sample. The method comprises the following steps: providing the biological sample comprising the target analyte on the sample area of the analysis array as described above; generating an image of the biological sample and determining the position of the biological sample with reference to the attachment zones of the analysis array; permeabilising the biological sample; determining the sequence of at least the location oligonucleotides bound to the affinity reagents; and assigning the bound target analyte to a part of the biological sample based on the sequence of the location oligonucleotides and the determined position of the biological sample on the analysis array. This enables capturing the target analyte on the analysis array and determining the location of target analyte on the analysis array and the biological sample based on a sequencing readout of the location oligonucleotide.

The image may in particular be generated by means of a microscope, in particular by a light microscope, by a fluorescence microscope, by a confocal light microscope - in particular a STED (stimulated emission depletion) microscope - or by a super-resolution light microscope applying a localisation technology such as PALM (photo-activated localization microscopy), STORM (stochastic optical reconstruction microscopy), GSDIM (ground state depletion microscopy followed by individual molecule return), or a technology according to SIM (spatially modulated illumination). In order to identify the attachment zones within the image, the attachment zones may preferably be marked by markers, such as fluorophores. For example, the markers of one attachment zone may be orientated relative to the affinity reagents of the particular attachment zone such that the affinity reagents of that attachment zone may be identified in an image of the analysis array. The step of permeabilising, in particular, enables binding of the target analyte of the biological sample to the respective affinity reagent. The step of permeabilising may in particular include lysing the biological sample, preferably before completely lysing the biological sample. The step of determining the sequence preferably also includes determining the sequence of the identity oligonucleotides, especially in case several different affinity reagents are used. The sequencing may be carried out in-situ or the oligonucleotides to be sequenced may be removed from the analysis array prior to sequencing.

The step of assigning the bound target analyte to a part of the biological sample may further include identifying the bound target analyte based on the sequence of the identity oligonucleotides, especially in case several different affinity reagents are used. Thus, pairs of identity oligonucleotides and location oligonucleotides may be sequenced and used to identity the target analyte bound to a particular affinity reagent and to assign the identified target analyte to a particular part of the biological sample based on which attachment zone the particular affinity reagent is attached to.

Preferably, the method further comprises the step of detaching the affinity reagents from the sample area. In particular, this may be achieved by cleaving the first linker with the first cleavage method. In particular, this step is carried out prior to sequencing. This allows collecting, in particular, the location oligonucleotides and preferably also the identity oligonucleotides. These may then be sequenced separately.

Preferably, the method further comprises the step of collecting the affinity reagents to which a target analyte is bound. In particular, this may be carried out prior to sequencing, for example, by complementary strand synthesis. This enables specifically sequencing the location oligonucleotides and the identity oligonucleotides from the respective affinity reagents and thus, it can be determined to which target analytes the respective affinity reagents were bound and therefore those target analytes can be determined.

Preferably, the affinity reagents are collected by means of affinity chromatography. For example, the affinity chromatography may be based on affinity towards the target analytes. In particular, this enables separating the affinity reagents to which target analytes have bound, and respective location and identity oligonucleotide, from affinity reagents to which no target analytes have bound.

Preferably, after the step of collecting the affinity reagents, at least the location oligonucleotides, and preferably the identity oligonucleotides, are detached from the collected affinity reagents. In particular, this may be achieved by cleaving the second linker with the second cleavage method. In particular, this step is carried out prior to sequencing. This enables efficiently sequencing at least the location oligonucleotides.

Preferably, the step of permeabilising the biological sample comprises adding the third affinity reagent to the biological sample. In particular, the third affinity reagent is introducing into the biological sample. This enables the third affinity reagent to bind the respective target analyte.

Preferably, the second linker of the third affinity reagent is cleaved prior to determining the sequence of the identity oligonucleotides and/or the location oligonucleotides. In particular, priorto detaching the affinity reagents from the sample area and prior to synthesising complementary strands. This enables separating the identity oligonucleotides and complementary oligonucleotides of the third affinity reagents from the remaining third affinity reagents and efficient hybridisation of the complementary oligonucleotides to the capture oligonucleotides.

Preferably, after the step of cleaving the second linker and detaching the affinity reagents from the analysis array, comprising location and identity oligonucleotides, the affinity reagents, especially the location and identity oligonucleotides, may be purified prior to sequencing, for example, by gel electrophoresis or silica adsorption. This enables efficient sequencing of pairs of location and identity oligonucleotides.

Preferably, prior to the step of determining the sequence of the identity oligonucleotides and location oligonucleotides, complementary strands to at least the identity oligonucleotides and location oligonucleotides are synthesised. This enables robust sequencing of at least the identity and location oligonucleotides. In particular, strands complementary to the identity and location oligonucleotides are synthesised.

Preferably, after permeabilising the biological sample, the biological sample is removed from the analysis array and unbound target analytes are removed. Unbound target analytes are not bound to the affinity reagents. The unbound target analytes may be washed away, for example.

### Short Description of the Figures

Hereinafter, specific embodiments are described referring to the drawings, wherein:
- Figure 1: is a schematic top view of an analysis array comprising a sample area with a plurality of attachment zones,
- Figure 2: is a schematic side view of three attachment zones of an analysis array with affinity reagents according to a first embodiment,
- Figure 3: is a schematic side view of three attachment zones of an analysis array with affinity reagents according to a second embodiment,
- Figure 4: is a flow chart of a method for analysing a biological sample by means of the analysis array according to Fig. 2,
- Figure 5: is a flow chart of a method for analysing a biological sample by means of the analysis array according to Fig. 3,
- Figure 6: is a schematic side and top view of the analysis array according to Fig. 2 with a biological sample,
- Figure 7: is a schematic side view of the analysis array according to Fig. 2 and a schematic cutaway of a chromatography column,
- Figure 8: is a schematic side and top view of the analysis array according to Fig. 3 with the biological sample, and
- Figure 9: is a schematic side view of the analysis array according to Fig. 3.

### Detailed Description

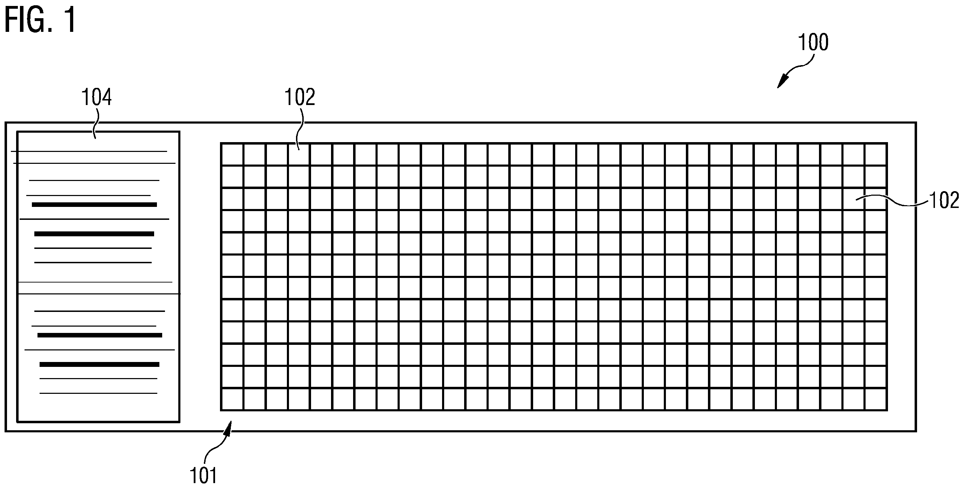

Figure 1 is a schematic top view of an analysis array 100 comprising a sample area 101 with a plurality of attachment zones 102. The attachment zones 102 are demarked by squares arranged in a 12 by 30 grid on the analysis array 100. The lines of the grid as shown in Figure 1 are merely shown as a visual aid and are not to scale. The attachment zones 102 may have other shapes, such as a circle or a polygon, in particular a hexagon, that fit into the squares of the grid as outlined in Figure 1. The attachment zones 102 may have a side length in a range from 5 µm to 100 µm, more preferably in a range from 10 µm to 50 µm, even more preferably in a range from 15 µm to 40 µm.

In order to identify the attachment zones 102 in an optical readout such as an image of the sample area of the analysis array 100, they may be marked by optically detectable moieties, such as fluorophores.

A biological sample comprising at least one target analyte may be placed on the sample area 101 with the attachment zones 102. The location of the biological sample on the sample area 101 may be determined relative to the attachment zones 102 based on the optically detectable moieties that mark the attachment zones 102.

The analysis array 100 may optionally comprise a label 104, such as an optically readable barcode, that enables identifying the particular analysis array 100 and a respective biological sample from a plurality of analysis arrays.

The analysis array 100 may be a microscope slide, in particular, a glass slide, on which the attachment zones 102 and the label 104 are arranged.

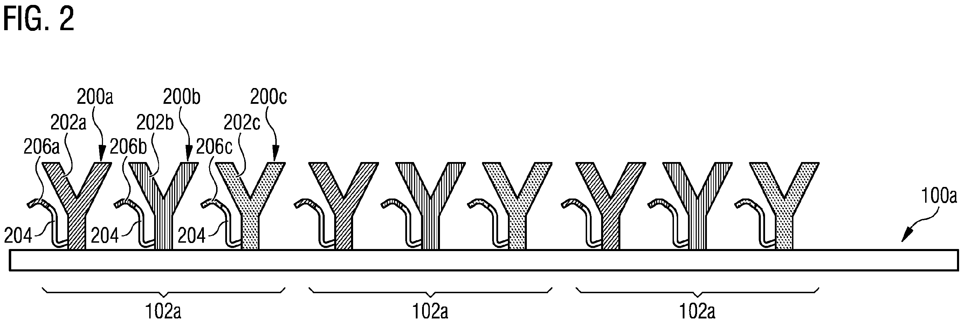

Figure 2 is a schematic side view of three attachment zones 102a of an analysis array 100a with affinity reagents according to a first embodiment.

The attachment zones 102a each comprise affinity reagents 200a, 200b, 200c that are configured to directly bind a target analyte. Each affinity reagent 200a, 200b, 200c specifically binds a different target analyte. Thus, the different target analytes may attach to one of the affinity reagents 200a, 200b, 200c in each of the attachment zones 102a. Each of the attachment zones 102a may comprise a plurality of each the affinity reagents 200a, 200b, 200c.

The affinity reagents 200a, 200b, 200c each comprise an antibody 202a, 202b, 202c with which the affinity reagents 200a, 200b, 200c specifically bind to the respective target analyte. Alternatively to the antibodies 202a, 202b, 202c, antibody fragments or aptamers may be used.

The affinity reagents 200a, 200b, 200c, in particular, the antibodies 202a, 202b, 202c are attached to the respective attachment zone 102a via a first linker (not shown) cleavable by a first cleavage method. This enables detaching and removing the affinity reagents 200a, 200b, 200c from the attachment zones 102a of the analysis array 100a. The cleavage of the first linker is preferably irreversible. An example of a first linker and first cleavage method is an oligonucleotide linker cleavable by a chemical agent, a light induced reaction or a specific restriction enzyme.

The affinity reagents 200a, 200b, 200c each comprise a location oligonucleotide 204. The location oligonucleotide 204 has a sequence that is unique to the particular one of the attachment zones 102a that the affinity reagents 200a, 200b, 200c are attached to.

Further, the affinity reagents 200a, 200b, 200c each comprise an identity oligonucleotide 206a, 206b, 206c. The identity oligonucleotide 206a, 206b, 206c is unique to the antibody 202a, 202b, 202c of the respective affinity reagent 200a, 200b, 200c.

The location oligonucleotides 204 and the identity oligonucleotides 206a, 206b, 206c are attached to the antibody 202a, 202b, 202c of the respective affinity reagent 200a, 200b, 200c. In particular, each pair of location oligonucleotide 204 and the identity oligonucleotides 206a, 206b, 206c is a single continuous nucleotide strand. The pairs of location oligonucleotides 204 and identity oligonucleotides 206a, 206b, 206c may be attached to the antibody 202a, 202b, 202c of the respective affinity reagent 200a, 200b, 200c via a second linker (not shown) cleavable by a second cleavage method. The cleavage of the second linker is preferably irreversible. An example of a second linker and second cleavage method is an oligonucleotide linker cleavable by a specific restriction enzyme a chemical agent or a light induced reaction. The second cleavage method is different to the first cleavage method, e.g. by different enzymes, different chemical agents or different wavelenghts of light which induce the cleavage, or combinations thereof.

Thus, the location oligonucleotide 204 and the identity oligonucleotide 206a, 206b, 206c of a particular one of the affinity reagents 200a, 200b, 200c allows the identification of the antibody 202a, 202b, 202c of the affinity reagent 200a, 200b, 200c and the attachment zone 102a, that the particular affinity reagent 200a, 200b, 200c is attached to.

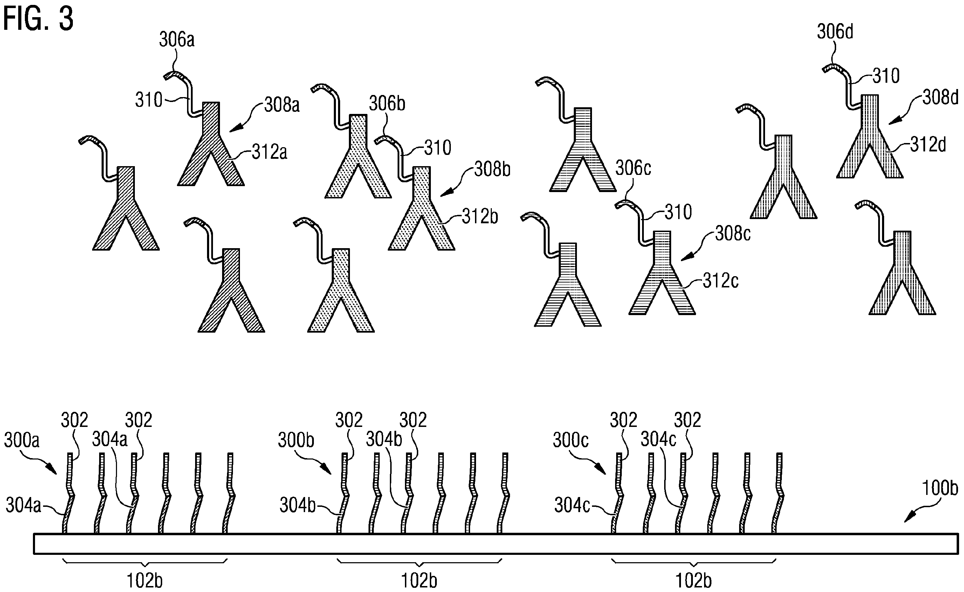

Figure 3 is a schematic side view of three attachment zones 102b of an analysis array 100b with affinity reagents 300a, 300b, 300c according to a second embodiment.

The affinity reagents 300a, 300b, 300c each comprise a capture oligonucleotide 302 and a respective location oligonucleotide 304a, 304b, 304c. The affinity reagents 300a, 300b, 300c are attached to the respective attachment zone 102b via a first linker (not shown) cleavable by a first cleavage method. This enables detaching and removing the affinity reagents 300a, 300b, 300c from the attachment zones 102b of the analysis array 100b. The cleavage of the first linker is preferably irreversible. An example of a first linker and first cleavage method is an oligonucleotide linker cleavable by a specific restriction enzyme.

The location oligonucleotide 304a, 304b, 304c have a respective sequence that is unique to the particular one of the attachment zones 102b that the corresponding affinity reagent 300a, 300b, 300c is attached to.

Furthermore, third affinity reagents 308a, 308b, 308c, 308d are provided that comprise identity oligonucleotides 306a, 306b, 306c, 306d and complementary oligonucleotides 310 that are complementary to the capture oligonucleotide 302 of the affinity reagents 300a, 300b, 300c. Further, the third affinity reagents each comprise an antibody 312a, 312b, 312c, 312d. Each antibody 312a, 312b, 312c, 312d specifically binds to a different target analyte in the biological sample. Alternatively to the antibody, an aptamer or an antibody fragment may be used.

The identity oligonucleotides 306a, 306b, 306c, 306d are unique to the antibody 312a, 312b, 312c, 312d of the respective third affinity reagent 308a, 308b, 308c, 308d.

Since the complementary oligonucleotides 310 are complementary to the capture oligonucleotides 302, the third affinity reagents 308a, 308b, 308c, 308d may bind to the affinity reagents 300a, 300b, 300c. Since the capture oligonucleotides 302 of all the affinity reagents 300a, 300b, 300c are the same, the third affinity reagents 308a, 308b, 308c, 308d may bind to all the affinity reagents 300a, 300b, 300c.

The complementary oligonucleotide 310 and the identity oligonucleotide 306a, 306b, 306c, 306d of each third affinity reagents 308a, 308b, 308c, 308d is a single continuous nucleotide strand that is attached to the respective antibody 312a, 312b, 312c, 312d via a second linker (not shown) cleavable by second cleavage method. The cleavage of the second linker is preferably irreversible. An example of a second linker and second cleavage method is an oligonucleotide linker cleavable by a specific restriction enzyme.

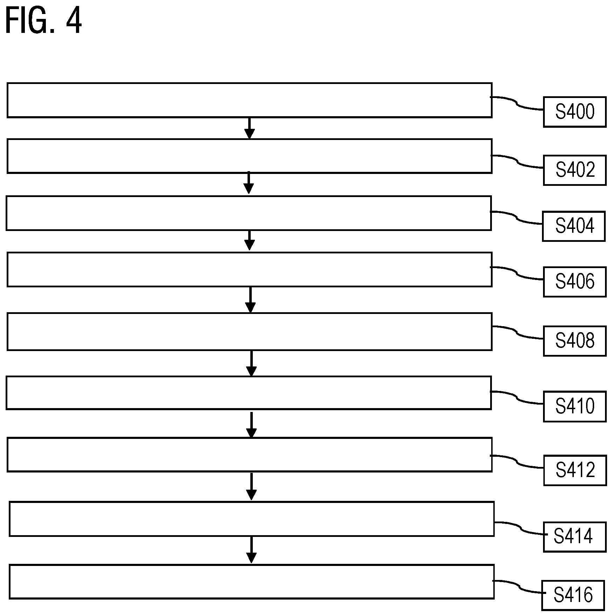

Figure 4 is a flow chart of a method for analysing a biological sample by means of the analysis array 100a with attachment zones 102a according to Fig. 2.

The method starts in step S400. In step S402, the biological sample is provided on the analysis array 100a, especially in a sample area comprising the attachment zones 102a.

In step S404, the biological sample is imaged on the analysis array 100a and the position of the biological sample on the analysis array 100a is determined relative to the attachment zones 102a. The attachment zones 102a may be marked with optically detectable markers in order to identify them in the image of the analysis array 100a with the biological sample.

In step S406, the biological sample is permeabilised in order to enable entry and/or exit of substances from the biological sample. Specifically, the target analytes may diffuse out of the biological sample onto the analysis array 100a and bind to the affinity reagents 200a, 200b, 200c of the respective attachment zone 102a. Generally, the target analytes bind to one of the affinity reagents 200a, 200b, 200c that is in one of the attachment zones 102a that is in close spatial proximity to the original location of the target analyte within the biological sample.

In step S408, the first linker is cleaved by the first cleavage method. This detaches all affinity reagents 200a, 200b, 200c from the analysis array 100a. At least some of the affinity reagents 200a, 200b, 200c have their respective target analytes bound.

In step S410, the affinity reagents 200a, 200b, 200c to which a target analyte is bound are separated or collected. This may be done by affinity chromatography, which binds all target analytes at a different epitope than the affinity reagents 200a, 200b, 200c and therefore enables specific collection of target analytes and their affinity reagents 200a, 200b, 200c.

In step S412, once the affinity reagents 200a, 200b, 200c with bound target analytes are separated from the affinity reagents 200a, 200b, 200c without bound target analytes, the second linker is cleaved and the location oligonucleotide 204 and the identity oligonucleotide 206a, 206b, 206c pairs of the affinity reagents 200a, 200b, 200c with bound target analytes separated in step S410 are collected. These pairs of oligonucleotides give an indication of the identity of those target analytes that were originally in the biological sample and their location within the biological sample.

In step S414, the location and identity oligonucleotide pairs are sequenced and based on the sequence information the identity of the target analytes in the biological sample and the location of the affinity reagents 200a, 200b, 200c they were capture with are determined. Further, based on the determined location of the biological sample relative to the attachment zones 102a on the sample area 101 of the analysis array 100a, a particular target analyte captured with one of the affinity reagents 200a, 200b, 200c of a particular one of the attachment zones 102a may be assigned to a location in the biological sample that is in close spatial proximity to that particular attachment zone 102a. Method ends in step S416.

Alternatively to the specific affinity chromatography in step S410, a cascade of affinity chromatography columns and chromatography steps may be used. In this case each column and chromatography step may be specific to one of the target analytes. Thus, the eluate of each chromatography column contains only affinity reagents 200a, 200b, 200c specific to one of the target analytes. In this case, no identity oligonucleotide 206a, 206b, 206c is needed, since the affinity reagents 200a, 200b, 200c are separated according to their identity by the affinity chromatography and for each identity the location oligonucleotides 204 are sequenced separately.

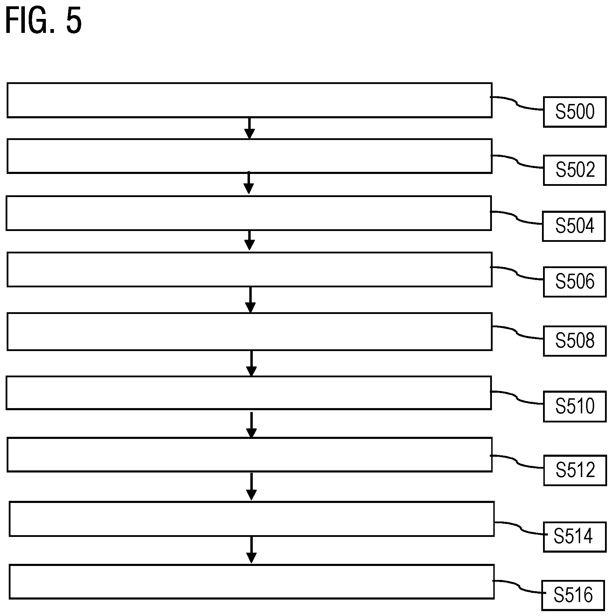

Figure 5 is a flow chart of a method for analysing a biological sample by means of the analysis array 100b with attachment zones 102b according to Fig. 3.

The method starts in step S500. In step S502, the biological sample is provided on the analysis array 100b, especially in a sample area comprising the attachment zones 102b.

In step S504, the biological sample is imaged on the analysis array 100b and the position of the biological sample on the analysis array 100b is determined relative to the attachment zones 102b. The attachment zones 102b may be marked with optically detectable markers in order to identify them in the image of the analysis array 100b with the biological sample.

In step S506, the biological sample is permeabilised in order to enable entry and/or exit of substances from the biological sample. Further, the third affinity reagents 308a, 308b, 308c, 308d are added to the biological sample. This enables binding of the third affinity reagents 308a, 308b, 308c, 308d, in particular, the respective antibodies 312a, 312b, 312c, 312d, to the target analytes within the biological sample. Subsequently, any unbound third affinity reagents 308a, 308b, 308c, 308d may be washed out of the biological sample.

In step S508 the second linker of the remaining third affinity reagents 308a, 308b, 308c, 308d with bound target analytes is cleaved with the second cleavage method. This enables the complementary oligonucleotides 310 together with the respective identity oligonucleotides 306a, 306b, 306c, 306d to bind to the affinity reagent 304a, 304b, 304c, in particular, to the capture oligonucleotides 302. Generally, the complementary oligonucleotides 310 bind to one of the affinity reagents 304a, 304b, 304c that is in one of the attachment zones 102b that is in close spatial proximity to the original location of the target analyte within the biological sample.

In step S510, complementary strands to the identity oligonucleotides 306a, 306b, 306c, 306d and location oligonucleotides 304a, 304b, 304c bound to the capture oligonucleotides 302 are synthesised.

In step S512, the first linker is cleaved by the first cleavage method. This detaches the synthesised constructs of step S510 from the analysis array 100b, in particular from the attachment zones 102b. Alternatively, the sequencing of the identity oligonucleotides 306a, 306b, 306c, 306d and location oligonucleotides 304a, 304b, 304c may be performed while the identity oligonucleotides 306a, 306b, 306c, 306d and location oligonucleotides 304a, 304b, 304c are still attached to the sample area 101 or to the analysis array 100b.

In step S514, the identity oligonucleotides 306a, 306b, 306c, 306d and location oligonucleotides 304a, 304b, 304c detached in step S512 are sequenced. Based on the sequence information, the presence and location of target analytes in the biological sample are determined. This is based on the determined location of the biological sample relative to the attachment zones 102b on the sample area of the analysis array 100b as well as the particular attachment zone 102b that the affinity reagent 300a, 300b, 300c is in to which the target analyte was in close spatial proximity when it bound to the third affinity reagent 308a, 308b, 308c, 308d. Method ends in step S516.

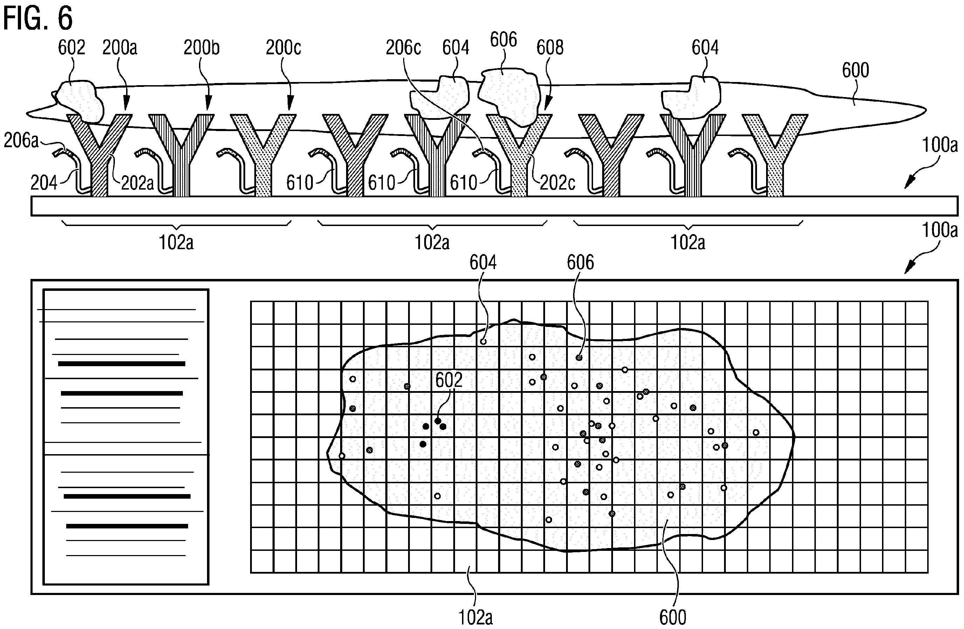

Figure 6 is a schematic side and top view of the analysis array 100a with a biological sample 600. The biological sample 600 comprises several target analytes 602, 604, 606 and is arranged on the sample area 101 of the analysis array 100a. The antibody 202a of the affinity reagent 200a specifically binds the target analyte 602. Thus, after permeabilising the biological sample 600 (step S406), the target analyte 602 binds to the affinity reagent 200a. The remaining target analytes 604, 606 may bind to further affinity reagents. For example, the target analyte 606 may bind specifically to the antibody 202c of an affinity reagent 608 attached to a different one of the attachment zones 102a that is in close proximity to the position of the target analyte 606 in the biological sample 600.

The location oligonucleotide 204 of the affinity reagent 200a and a location oligonucleotide 610 of the affinity reagent 608 each have a sequence that is unique to the particular one attachment zone 102a that the respective affinity reagent 200a, 608 is attached to. Similarly, the identity oligonucleotide 206a of the affinity reagent 200a is unique to the antibody 202a, whereas the identity oligonucleotide 206c of the affinity reagent 608 is unique to the antibody 202c.

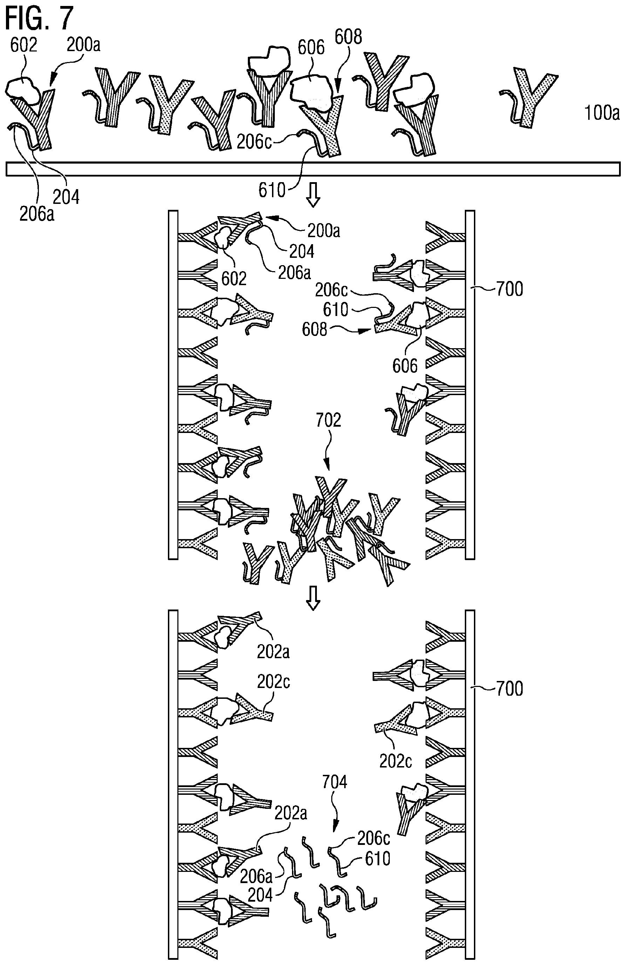

Figure 7 is a schematic side view of the analysis array 100a and a schematic cutaway of a chromatography column 700.

In the top view of Fig. 7, step S408 is depicted wherein the first linker is cleaved by the first cleavage method. This detaches all affinity reagents 200a, 200b, 200c, 608 from the analysis array 100a. At least some of the affinity reagents 200a, 200b, 200c, 608 have their respective target analytes 602, 604, 606 bound.

In the middle view, step S410 is depicted wherein the affinity reagents 200a, 200b, 200c, 608 to which a target analyte 602, 604, 606 is bound are separated or collected by means of an affinity chromatography column 700, which binds all target analytes 602, 604, 606 together with the respective affinity reagents 200a, 200b, 200c, 608 and therefore enables specific collection of target analytes 602, 604, 606 and their affinity reagents 200a, 200b, 200c, 608. The affinity reagents 200a, 608 are then separated from affinity reagents 702 that have no target analyte bound.

In the bottom view, step S412 is depicted wherein the second linker is cleaved and the location oligonucleotide 204, 610 and the identity oligonucleotide 206a, 206b, 206c pairs 704 of the affinity reagents 200a, 200b, 200c, 608 with bound target analytes 602, 604, 606 are collected. These pairs 704 of oligonucleotides, in particular their sequences, give an indication of the identity of those target analytes 602, 604, 606 that were originally in the biological sample 600 and their location within the biological sample 600.

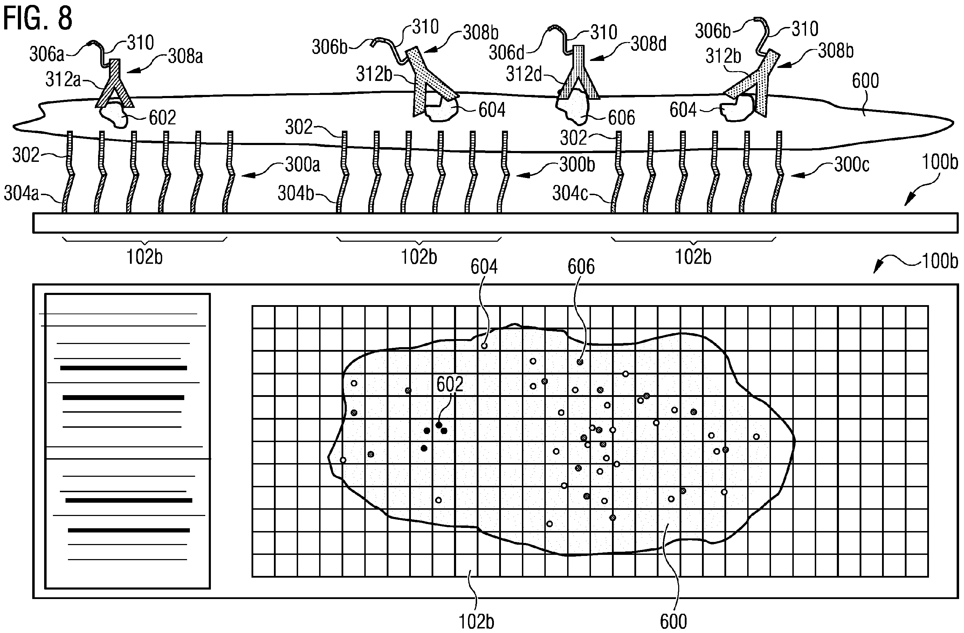

Figure 8 is a schematic side and top view of the analysis array 100b with the biological sample 600. The biological sample 600 comprises several target analytes 602, 604, 606 and is arranged on the sample area of the analysis array 100b. The third affinity reagents 308a, 308b, 308c, 308d are added to the permeabilised biological sample 600 (step S506) and bound to their respective target analyte 602, 604, 606. Exemplarily, the antibody 312a of third affinity reagent 308a is bound to the target analyte 602.

Subsequently, any unbound third affinity reagents 308a, 308b, 308c, 308d may be washed out of the biological sample, preferably after a predeterminable reaction time in which a lot of or most of or all of the third affinity reagents 308a, 308b, 308c, 308d might have attached to the target analytes 602, 604, 606.

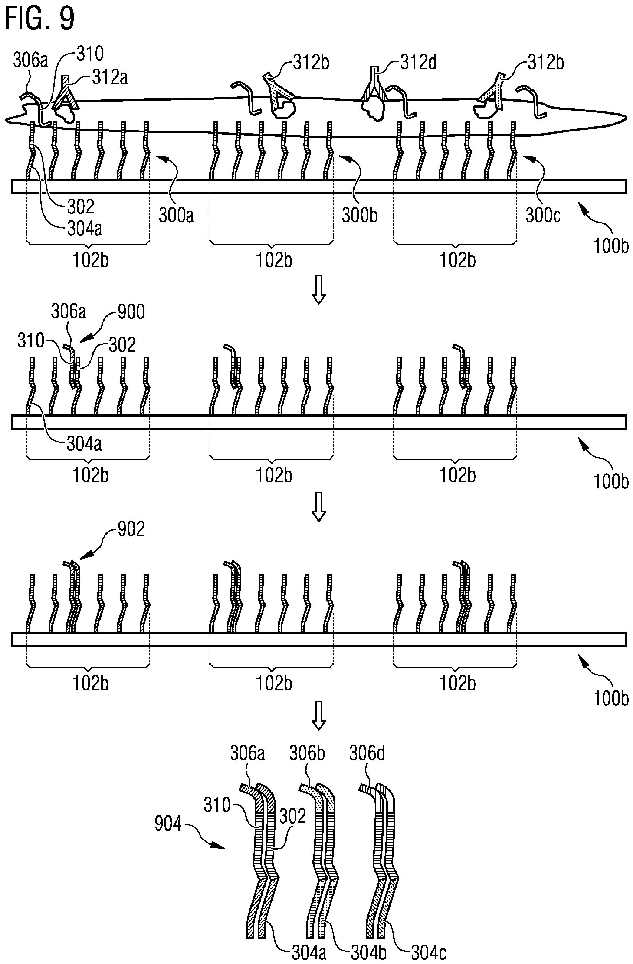

Figure 9 is a schematic side view of the analysis array 100b at various steps of the method described in connection with Fig. 5.

In the top and second from top view of Fig. 9, step S508 is depicted wherein the second linker of the remaining third affinity reagents 308a, 308b, 308c, 308d with bound target analytes 602, 604, 606 is cleaved with the second cleavage method. This enables the complementary oligonucleotides 310 together with the respective identity oligonucleotides 306a, 306b, 306c, 306d to bind to the affinity reagent 304a, 304b, 304c, in particular, to the capture oligonucleotides 302, to form hybridised capture oligonucleotides 900, see Fig. 5, middle view. Generally, the complementary oligonucleotides 310 bind to one of the affinity reagents 304a, 304b, 304c that is in one of the attachment zones 102b that is in close spatial proximity to the original location of the target analyte 602, 604, 606 within the biological sample 600.

In the third from top view, step S510 is depicted where complementary strands to the identity oligonucleotides 306a, 306b, 306c, 306d and location oligonucleotides 304a, 304b, 304c bound to the capture oligonucleotides 302 are synthesised. This forms sequencing constructs 902.

In the bottom view, step S512 is depicted wherein the first linker is cleaved by the first cleavage method. This detaches the synthesised constructs 902 of step S510 from the analysis array 100b, in particular from the attachment zones 102b, to give free sequencing constructs 904 that may be sequenced.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 100a, 100b: Analysis array
- 101: Sample area
- 102, 102a, 102b: Attachment zone
- 104: Label
- 200a, 200b, 200c, 300a, 300b, 300c, 608: Affinity reagent
- 202a, 202b, 202c: Antibody of affinity reagent
- 204, 304a, 304b, 304c, 610: Location oligonucleotide
- 206a, 206b, 206c, 306a, 306b, 306c, 306d: Identity oligonucleotide
- 302: Capture oligonucleotide
- 308a, 308b, 308c, 308d: Third affinity reagent
- 310: Complementary oligonucleotide
- 312a, 312b, 312c, 312d: Antibody of third affinity reagent
- 600: Biological sample
- 602, 604, 606: Target analyte
- 700: Chromatography column
- 702: Affinity reagent with no bound target analyte
- 704: Pair of location and identity oligonucleotide
- 900: Hybridised capture oligonucleotide
- 902: Sequencing construct
- 904: Free sequencing construct

## Claims

1. An analysis array (100, 100a, 100b) for analysing a biological sample (600) with a target analyte (602, 604, 606), the target analyte is an amino acid structure or a lipid, the analysis array comprising:
a sample area (101) configured to receive the biological sample (600), and
a plurality of affinity reagents (200a, 200b, 200c, 300a, 300b, 300c) configured to directly or indirectly bind the target analyte (602, 604, 606),
wherein the sample area (101) comprises a plurality of attachment zones (102, 102a, 102b) for attachment of the affinity reagents (200a, 200b, 200c, 300a, 300b, 300c) to the sample area (101),
wherein each affinity reagent (200a, 200b, 200c, 300a, 300b, 300c) comprises a location oligonucleotide (204, 304a, 304b, 304c, 610), and
wherein the location oligonucleotide (204, 304a, 304b, 304c, 610) is unique to the attachment zone (102, 102a, 102b) the affinity reagent (200a, 200b, 200c, 300a, 300b, 300c) is attached to.

2. The analysis array according to claim 1, wherein the affinity reagents (200a, 200b, 200c, 300a, 300b, 300c) are attached to the attachment zones (102, 102a, 102b) via a first linker.

3. The analysis array according to any one of the preceding claims, wherein the affinity reagents (200a, 200b, 200c, 300a, 300b, 300c) comprise an amino acid structure or a nucleic acid structure configured to bind the target analyte (602, 604, 606).

4. The analysis array according to any one of the preceding claims, wherein each affinity reagent (200a, 200b, 200c, 300a, 300b, 300c) comprises an identity oligonucleotide (206, 206b, 206c, 306a, 306b, 306c, 306d) unique to the affinity reagent (200a, 200b, 200c, 300a, 300b, 300c).

5. The analysis array according to any one of the preceding claims, comprising a plurality of second affinity reagents (200a, 200b, 200c) configured to bind a second target analyte (602, 604, 606), wherein each second affinity reagent (200a, 200b, 200c) comprises a second location oligonucleotide (204, 610) unique to the attachment zone (102, 102a, 102b) the second affinity reagent (200a, 200b, 200c) is attached to, and wherein each second affinity reagent (200a, 200b, 200c) comprises a second identity oligonucleotide (206, 206b, 206c) unique to the second affinity reagent (200a, 200b, 200c).

6. The analysis array according to any one of the preceding claims, wherein the location oligonucleotides (204, 304a, 304b, 304c, 610) are attached to the affinity reagents (200a, 200b, 200c, 300a, 300b, 300c) via a second linker.

7. The analysis array according to any one of preceding claims 1 to 3, wherein the affinity reagents (300a, 300b, 300c) comprise a capture oligonucleotide (302) and the capture oligonucleotide (302) is configured to bind the target analyte (602, 604, 606) via a third affinity reagent (308a, 308b, 308c, 308d) configured to directly bind the target analyte (602, 604, 606) of the biological sample (600), wherein the third affinity reagent (308a, 308b, 308c, 308d) comprises an identity oligonucleotide (306a, 306b, 306c, 306d) unique to the third affinity reagent (308a, 308b, 308c, 308d), and an oligonucleotide (310) complementary to the capture oligonucleotide (302) of the affinity reagent.

8. A method for analysing a biological sample (600) comprising the following steps:
providing the biological sample (600) comprising a target analyte (602, 604, 606) on the sample area (101) of the analysis array (100, 100a, 100b) according to one of the preceding claims,
generating an image of the biological sample (600) and determining a position of the biological sample (600) with reference to the attachment zones (102, 102a, 102b) of the analysis array (100, 100a, 100b),
permeabilising the biological sample (600),
determining a sequence of at least the location oligonucleotides (204, 304a, 304b, 304c) bound to the affinity reagents (200a, 200b, 200c, 300a, 300b, 300c), and
assigning the bound target analyte (602, 604, 606) to a part of the biological sample (600) based on the sequence of the location oligonucleotides (204, 304a, 304b, 304c) and the determined position of the biological sample (600) on the analysis array (602, 604, 606).

9. The method according to claim 8, further comprising the step of detaching the affinity reagents (200a, 200b, 200c, 300a, 300b, 300c) from the sample area (101).

10. The method according to any one of the claims 8 or 9, further comprising the step of collecting the affinity reagents (200a, 200b, 200c, 300a, 300b, 300c) to which a target analyte (602, 604, 606) is bound.

11. The method according to claim 10, wherein the affinity reagents (200a, 200b, 200c, 300a, 300b, 300c) are collected by means of affinity chromatography.

12. The method according to any one of the claims 10 or 11, wherein after collecting the affinity reagents (200a, 200b, 200c, 300a, 300b, 300c), the location oligonucleotides (204, 304a, 304b, 304c) are detached from the collected affinity reagents (200a, 200b, 200c, 300a, 300b, 300c).

13. The method according to any one of the claims 8 or 9, wherein the step of permeabilising the biological sample (600) comprises adding the third affinity reagent (308a, 308b, 308c, 308d) to the biological sample (600).

14. The method according to claim 13, wherein the second linker of the affinity reagent (200a, 200b, 200c, 300a, 300b, 300c) is cleaved prior to determining the sequence of the identity oligonucleotides (206, 206b, 206c, 306a, 306b, 306c, 306d) and the location oligonucleotides (204, 304a, 304b, 304c, 610).

15. The method according to any one of the claims 13 or 14, wherein prior to the step of determining the sequence of the identity oligonucleotides (206, 206b, 206c, 306a, 306b, 306c, 306d) and location oligonucleotides (204, 304a, 304b, 304c, 610), complementary strands to at least the identity oligonucleotides (206, 206b, 206c, 306a, 306b, 306c, 306d) and location oligonucleotides (204, 304a, 304b, 304c, 610) are synthesised.

16. The method according to any one of the claims, wherein after permeabilising the biological sample (600), the biological sample (600) is removed from the analysis array (100, 100a, 100b) and unbound target analytes (602, 604, 606) are removed.
